# EUROPEAN PATENT APPLICATION

(11) **EP 4 358 646 A1**
(43) Date of publication of application: **24.04.2024**
(21) Application number: 22824184.0
(22) Date of filing: 14.06.2022
(51) Int. Cl.: H05B 45/00, A61B 5/16

(54) **LIGHTING SYSTEM HAVING MULTI-SPECTRAL RHYTHM LIGHTING SCENARIO, AND LIGHTING METHOD THEREOF**

(30) Priority: 15.06.2021 US 202163210686 P
(71) Applicant: Lin, Lawrence, Taoyuan City, Taiwan 330010 (TW)
(72) Inventor: CHANG, Chih Hung, New Taipei 242073, Taiwan (TW); LIN, Lawrence, Taoyuan City 330010, Taiwan (TW)
(74) Representative: Lang, Christian
(86) International application number: PCT/CN2022/098583
(87) International publication number: WO 2022/262705

(57) **Abstract**

A lighting system with a multispectral circadian rhythm lighting scenario, which performs lighting by the following procedures, the procedures include: downloading the multispectral circadian rhythm lighting scenario database from a portable communication device to the cloud through the Internet; the portable communication device selects a multispectral lighting parameter and a circadian rhythm parameter from the multispectral circadian rhythm lighting scenario database to start the light-emitting device to control the illuminator to perform lighting; after the blood or saliva test of the light control subjects, the psychological stress index values of the illuminator were obtained; the portable communication device or the management control module transmits the circadian rhythm parameters and psychological stress index values to the cloud.

## Description

### FIELD OF THE INVENTION

The present invention relates to a system and a method for establishing a multispectral circadian rhythm lighting scenario database, particularly relates to a lighting process through multispectral lighting parameters and circadian rhythm parameters to adjust the circadian rhythm of the human body.

### BACKGROUND OF THE INVENTION

Humans are animals with changeable emotions, they will have different emotional reactions with the individual's psychological state, such as excitement, amusement, anger, disgust, fear, happiness, sadness, serene, neutral. When negative emotions (such as anger, disgust and fear) cannot be resolved in time, they will cause psychological damage or trauma to the human body, and finally evolve into mental illness. Therefore, how to timely provide an emotional resolution or relief or treatment system that can meet the needs of users has broad business opportunities in today's society, full of high competition and high pressure at any time.

In modern medical equipment, the changes of hemodynamics caused by neuronal activity can be measured by functional Magnetic Resonance Imaging (fMRI) system. Due to the non-invasiveness of fMRI and its low radiation exposure, fMRI is currently mainly used in the study of human and animal brain or spinal cord. At the same time, the tester can also be checked by the electroencephalogram of the EEG, and the emotions can be stimulated in the same way, and the responses of different emotions can be seen, for example, the brainwave patterns of fear and happiness can be seen significantly different. Among them, when observing the response of a certain emotion under fMRI and electroencephalograph (EEG), for example, under happy emotion (which can be induced by pictures and matched with facial emotion recognition), fMRI was used to observe the blood oxygen-level dependent (BOLD) contrast response and found that in the medial prefrontal cortex (Mpfc), there were significantly more responses to the corresponding emotions (anger and fear). In contrast, for example, in the case of fear and anger, fMRI was used to observe the blood oxygen-level dependent (BOLD) contrast response, and it was found that it was significantly reflected in the amygdala region, showing that the two kinds of emotions have different response regions in the brain. Therefore, the blood oxygen-level dependent (BOLD) contrast response of different regions in the brain can be used to clearly determine which emotion the tester is currently in. In addition, if the electroencephalogram of the EEG is used to examine the measurement tester, and the emotions are stimulated in the same way, it can be seen that the brainwave patterns of fear and happiness are significantly different. Therefore, you can also judge what kind of emotion the tester is currently in through the brainwave patterns of different reactions. According to the above, for the functional Magnetic Resonance Imaging (fMRI) system, emotions are distinguished by different blood oxygen-level dependent (BOLD) contrast reactions, while for the electroencephalograph (EEG), emotions are distinguished by different brainwave patterns. It is obvious that the methods used to judge the emotion of the tester and the contents recorded are completely different. Therefore, in terms of current science and technology, the brainwave patterns of electroencephalograph (EEG) cannot replace the blood oxygen-level dependent (BOLD) contrast response of functional Magnetic Resonance Imaging (fMRI) for the test results of the same emotion of the same tester.

The above discussion on emotion judgment by functional Magnetic Resonance Imaging (fMRI) system and electroencephalograph (EEG) is that fMRI system is very expensive and huge, so it cannot be used in commercial systems and methods of human centric lighting. Similarly, if only the brainwave patterns of the electroencephalograph (EEG) are used to judge the mood of the tester, it may be encountered that the brainwave patterns of different testers for different emotions may be different. Therefore, at present, it is impossible to use the blood oxygen-level dependent (BOLD) contrast response of functional magnetic resonance imaging system (fMRI) alone, or the brain wave mode of electroencephalograph (EEG) alone to construct a commercial human centric lighting method and system through the editing of spectral recipe.

In addition, humans also have obvious biological circadian rhythms, known as circadian rhythms ("biological clocks"), which are controlled by the body's physiological clocks, and the control inference of these physiological clocks is affected by light. Later, in the study of the effect of light on physiological stimulation, it has been found that the light source with high color temperature at night inhibits the secretion of melatonin more than the light source with low color temperature. In addition, from the relatively new research, it is found that the human eye is highly related to the biological effect. Therefore, it further proves the relationship between light source and melatonin secretion and physiological effect. Therefore, if the human body works at night, it will make the living body in the circadian rhythm at night, so it is unable to refresh. It is necessary to provide spectral components with daytime light sources so that the human body can provide work efficiency at night.

### SUMMARY OF THE INVENTION

According to the above description, the present invention provides a lighting system with multispectral circadian rhythm lighting scenario, which is configured in a space and is composed of a portable communication device, a management control module, a cloud, an ambient light sensor and a light-emitting device. Its feature is that the lighting system performs lighting through the following procedures, wherein the procedures include:
acquiring the multispectral circadian rhythm lighting scenario database, which is downloaded from the portable communication device to the cloud through internet;
starting the lighting of the circadian rhythm lighting scenario, a portable communication device selects a multispectral lighting parameter and a circadian rhythm parameter from the multispectral circadian rhythm lighting scenario database to start the light-emitting device to perform an illumination on an illuminator;
performing adrenal cortisol test to obtain the psychological stress index value in the blood and saliva of the illuminator after the blood or saliva test of the illuminator;
establishing a multispectral circadian rhythm lighting scenario and psychological stress correlation database, and the portable communication device or the management control module transmits the circadian rhythm parameters and the psychological stress index values to the cloud.

The invention then provides a lighting system with a multispectral circadian rhythm lighting scenario, which is configured in a space and is composed of a portable communication device, a management control module, a cloud, an ambient light sensor and a light-emitting device. The feature is that the lighting system performs lighting through the following procedures, wherein the procedures include:
acquiring a multispectral circadian rhythm lighting scenario database, wherein the portable communication device downloads the multispectral light-emitting device cloud database through internet to the cloud;
starting the lighting of the circadian rhythm lighting scenario, the portable communication device selects a multispectral lighting parameter and a circadian rhythm parameter from the multispectral circadian rhythm lighting scenario database, transmitting the parameters to the ambient light sensor, and then the ambient light sensor actives the light-emitting device to perform an illumination on an illuminator;
performing adrenal cortisol test is to obtain the psychological stress index value in the blood and saliva of the illuminator after sampling and testing the blood or saliva of the illuminator;
establishing a multispectral circadian rhythm lighting scenario and psychological stress correlation database, and the portable communication device or the management control module transmits the circadian rhythm parameters and the psychological stress index values to the cloud.

In establishing the above lighting system with multispectral circadian rhythm lighting scenario, the present invention also includes circadian rhythm parameters, including circadian rhythm lighting parameters and circadian rhythm scenario parameters.

In the establishment of the above lighting system with multispectral circadian rhythm lighting scenario, the circadian rhythm lighting parameters include: Equivalent Melanopic Lux (EML), Circadian Action Factor (CAF), sunshine illuminance, color temperature change, etc.

In establishing the above lighting system with multispectral circadian rhythm lighting scenario, its characteristics also include: the circadian rhythm parameters include: the circadian rhythm scenario parameters are composed of the light from the direct lighting device and the indirect lighting device.

In the establishment of the above lighting system with multispectral circadian rhythm lighting scenario, its feature is that the direct lighting device is composed of a recessed lamp, a pendant lamp and a ceiling lamp.

In establishing the above lighting system with multispectral circadian rhythm lighting scenario, its feature is that indirect lighting is the light reflected by the lighting light of lamps through the wall.

In the establishment of the above lighting system with multispectral circadian rhythm lighting scenario, its characteristics are that the multispectral lighting parameters are the lighting parameters such as spectrum, light intensity, flicker frequency and color rendering (Ra) of the lamp group.

In establishing the above lighting system with multispectral circadian rhythm lighting scenario, the information in the established multispectral circadian rhythm lighting scenario database includes: multispectral lighting parameters and circadian rhythm parameters.

After the establishment of the above multispectral circadian rhythm lighting scenario database and lighting system, it can be confirmed that the lighting field end can provide effective multispectral lighting parameters and circadian rhythm parameters of the circadian rhythm lighting scenario, so that the invention can provide an effective lighting field end and lamp group of the circadian rhythm lighting scenario, so that the lighting field can be popularized and used commercially.

### BRIEF DESCRIPTION OF THE DRAWINGS

FIG.1a is the original data collection structure of the human centric lighting to physiological and emotional according to the present invention.
FIG.1b is the original data collection of the human centric lighting to physiological and emotional according to the present invention.
FIG.1c is the judgment flow for the response of human centric lighting to specific physiological and emotional according to the present invention.
FIG.2a is a method for establishing the grading of physiological and emotional responses of the brain wave diagram of human centric lighting.
FIG.2b is the lighting database that the invention constructs the user to carry out effective human centric lighting.
FIG.3 is the system frame diagram of the intelligent human centric lighting system of the invention.
FIG.4 is a method for constructing a human centric light environment platform of the present invention.
FIG.5 is a schematic diagram of indoor light distribution of the invention.
FIG.6 is a lighting system applied to a spectral circadian rhythm lighting scenario.
FIG.7 is a method for establishing a multispectral circadian rhythm lighting database.
FIG.8 is a method for establishing a database of correlation between multispectral circadian rhythm lighting and psychological stress indicators.
FIG.9 is a lighting method having a multispectral circadian rhythm lighting scenario.

### DETAILED DESCRIPTION OF THE PREFERRED EMBODIMENT

In the specification after the present invention, the functional magnetic resonance imaging system is referred to as "fMRI system", the electroencephalograph is referred to as "EEG", and the blood oxygen-level dependent comparison is referred to as "BOLD". In addition, in the embodiment of the color temperature test of the present invention, the test is carried out in units of every 100K. However, in order to avoid too lengthy description, in the following description, the so-called specific emotion refers to excitement or excitement, happiness, amusement, etc., and the corresponding specific emotional response will be explained with 3,000K, 4,000K and 5,700K as color temperature test examples, therefore, the present invention cannot be limited to the embodiments of the three color temperatures. At the same time, in order to make those in the technical field of the present invention fully understand the technical content, relevant embodiments and embodiments thereof are provided for explanation. In addition, when reading the embodiment provided by the present invention, please also refer to the schema and the following description, in which the shape and relative size of each component in the schema are only used to assist in understanding the content of the embodiment, not to limit the shape and relative size of each component.

The present invention uses fMRI system to understand the corresponding relationship between spectrum and emotion in the brain through physiological signal measurement method and formulates a set of preliminary mechanism of using light to affect human physiological and psychological reactions, because the brain imaging of fMRI system can judge which part of the human brain has hyperemia reaction when stimulated by light, it can also record BOLD reaction. This BOLD response to brain hyperemia is also known as the "rise of blood oxygen concentration dependent response". Therefore, according to the image recording data of brain hyperemia under various emotions of fMRI system and the response of "rise of blood oxygen-level dependent response", the present invention can accurately and objectively infer the physiological and emotional changes of the tester, and then take the physiological and emotional confirmed by fMRI system as the basis, further, EEG is used to record the changes of EEG to establish the correlation between them, in order to use the changes of EEG to replace the emotion judgment of fMRI system.

Therefore, the main purpose of the present invention is to enable the tester to record the BOLD response of the tester to the specific emotion after illuminating the tester during the test of specific emotion in the fMRI system, so as to screen out which specific "effective color temperature" can multiply the specific emotion, the color temperature of lighting is used as the "effective color temperature" corresponding to specific emotions. After that, the tester is illuminated with "effective color temperature", and the EEG is used to record the brainwave patterns under the stimulation of "effective color temperature", so that the specific brainwave patterns of the EEG is related to the specific BOLD response. After that, the user's emotional change can be assisted by the specific EEG mode of the EEG, in order to construct a set of intelligent human centric lighting system and its method that can be operated commercially, so as to solve the problem that expensive fMRI system must be used to execute intelligent human centric lighting system, which can reduce the operation cost and further meet the customized service demand.

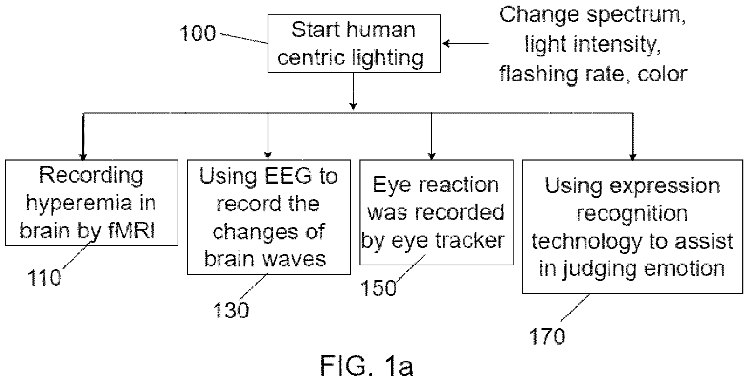

First, please refer to FIG. 1a, which is the original data collection framework of physiological and emotional due to human centric lighting of the present invention. As shown in FIG. 1a, starting the intelligent human centric lighting system 100 is in an environment where various adjustable lighting modules have been configured (e.g., a test space), and provides light signal lighting parameters such as spectrum, light intensity, flicker rate and color temperature that can be changed. For different target emotions, the present invention uses the fMRI compatible image interaction platform 110 formed by the fMRI system to guide the emotion of voice and image, and at the same time, it is matched with a specific effective spectrum to stimulate for 40 seconds. Observe the changes in the area where the blood oxygen-level changes in the tester's brain to verify whether the "effective color temperature" can significantly induce the tester's emotional response, and the details are as follows.

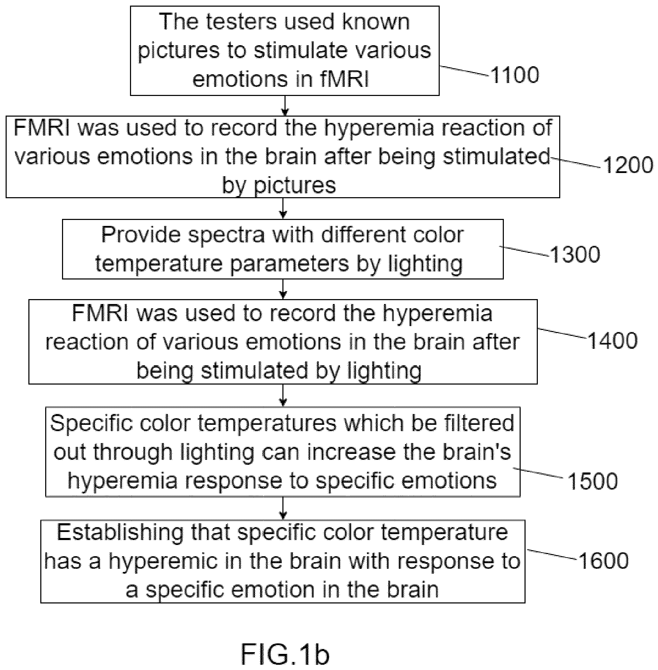

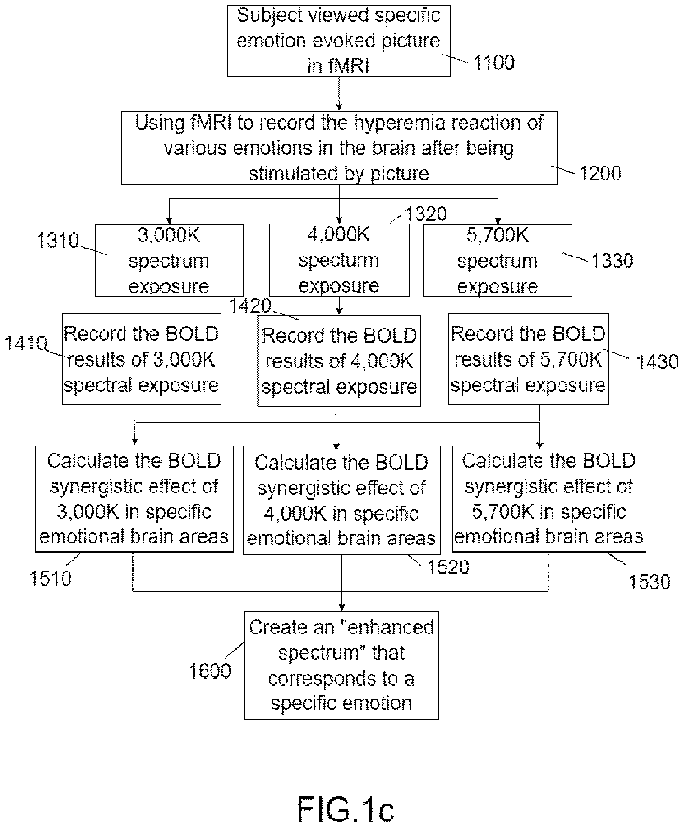

Next, please refer to FIG. 1b and FIG. 1c, wherein FIG. 1b is a flow chart of collecting original data of human centric lighting to physiological and emotional responses according to the present invention, and FIG. 1c is a judging flow of human centric lighting to specific physiological and emotional responses. As shown in step 1100 in FIG. 1b, each tester has been positioned on the compatible image interaction platform 110, and then each tester is guided to stimulate various emotions through known pictures. Afterwards, as shown in step 1200, the BOLD response in the brain of the tester's various emotions after being stimulated by the picture is recorded through the compatible image interaction platform 110. Next, as shown in step 1300, the tester is visually stimulated by irradiating light to provide spectrums with different color temperature parameters. For example: use LED lamps with electronic dimmers to provide spectrums with different color temperature parameters. In the embodiment of the present invention, nine groups of visual stimuli with different color temperatures, including 2,700K, 3,000K, 3,500K, 4,000K, 4,500K, 5,000K, 5,500K, 6,000K, and 6,500K, are provided. Among them, after completing 40 seconds of effective light irradiation and stimulation each time, you can choose to give testers one minute of invalid light source lighting (full spectrum non-flicker white light) to achieve the purpose of emotional relaxation. Further, it is also possible to choose to give the tester a 40 second counter effect light stimulation to observe whether the response in the area that responded to the original effective light stimulation decreased. In the embodiment of the present invention, after the compatible image interaction platform 110 has recorded the BOLD response in the brain of the tester's specific emotion after being stimulated by the picture, the tester is visually inspected by providing spectra with different color temperature parameters. The stimulus, as shown in step 1310 in FIG. 1c, is used to provide a spectrum with a color temperature of 3,000K, then, as shown in step 1320, to provide a spectrum with a color temperature of 4,000K, and finally, as shown in step 1330, to provide a color temperature for the 5,700K spectrum, the test subjects were visually stimulated.

Next, as shown in step 1400, the BOLD response of the test subject's brain after being stimulated by light is recorded through the compatible image interaction platform 110. In the embodiment of the present invention, after the tester is stimulated by lighting with different color temperature parameters, the compatible image interaction platform 110 records the BOLD response results with emotional response areas in the corresponding limbic system of the tester in sequence. Among them, the location of limbic system triggered by different emotions is different, and the above limbic system with emotional response in brain area is shown in Table 1 below.

**Table 1**

| Limbic system | Function |
|---|---|
| ACC | Emotion |
| Cuneus | Vision, Control bipolar disorder |
| Cerebellum | Balance, Coordination, Antidepressant & Anxiety |
| Insula Lobe | Affection, addiction |
| Lingual Gyrus | Logic, Vision , Antidepressant & Anxiety |
| Rolandic Operculum | Emotion Management, Gustatory Sense |
| Superior Frontal Gyrus | Working Memory, Self-consciousness, laugh |
| Middle Temporal Gyrus | Emotion Recognition, Creativity |
| MCC | Emotion |
| Pallidum | Affection, Motion, Cognition, Reward System |
| Temporal Pole | Affection, Language, Auditory |

The compatible image interaction platform 110 records the response result of BOLD in a specific area of the brain, and the response result is judged by calculating the area of these emotional response parts when the brain area has more limbic system with BOLD emotional response, for example: when there is a BOLD emotional response The larger the area of emotional response, the stronger the response to a specific physiological and emotional. In the embodiment of the present invention, as shown in step 1410 in FIG. 1c, it is used to record the reaction result of BOLD with a color temperature of 3,000K after spectral irradiation, and then, as shown in step 1420, it is used to record the color temperature of 4,000K. Finally, as shown in step 1430, the reaction result of BOLD after spectral irradiation is used to record the reaction result of BOLD after spectral irradiation with a color temperature of 5,700K. Among them, after the tester passes through the lighting procedure after the excitement is induced, the compatible image interaction platform 110 records the response results of BOLD in a specific area of the brain as shown in Table 2.

**Table 2**

| Excitement Index | | | |
|---|---|---|---|
| Limbic system | 3,000K | 4,000K | 5,700K |
| ACC | 0 | 0 | -30 |
| Cerebellum | 577 | 0 | -75 |
| Middle Temporal Gyrus | 0 | 99 | 0 |
| Superior Frontal Gyrus | 0 | 127 | 0 |
| Total T Score | 577 | 226 | -105 |

Among them, after the tester passes through the lighting procedure induced by happiness, the compatible image interaction platform 110 records the response results of BOLD in a specific area of the brain as shown in Table 3.

**Table 3**

| Happiness Index | | | |
|---|---|---|---|
| Limbic system | 3,000K | 4,000K | 5,700K |
| ACC | 0 | 0 | -162 |
| Cerebellum | 163 | 252 | 0 |
| Pallidum | 80 | 0 | 0 |
| Insula Lobe | 85 | 307 | 0 |
| Lingual Gyrus | 0 | 58 | 403 |
| Temporal Pole | 0 | 57 | 0 |
| Total T Score | 328 | 674 | 241 |

Among them, after the tester passes through the lighting procedure after the emotion-induced amusement, the compatible image interaction platform 110 records the response results of BOLD in a specific area of the brain as shown in Table 4.

**Table 4**

| Amusement Index | | | |
|---|---|---|---|
| Limbic system of brain | 3,000K | 4,000K | 5,700K |
| Lingual Gyrus | 0 | 279 | 327 |
| Cerebellum | 539 | 215 | 226 |
| Middle Temporal Gyrus | 0 | 0 | 90 |
| MCC | -83 | 32 | 0 |
| Total T Score | 456 | 526 | 643 |

Afterwards, as shown in step 1500, the results of the BOLD reaction that a specific color temperature can increase a specific emotion are screened by lighting, and the specific color temperature is called an "effective color temperature". In this embodiment, the BOLD response result of the emotional response area in the limbic system of the tester's corresponding brain is recorded, so as to summarize the stimulation effect of color temperature on the brain, as shown in Tables 1 to 3 shown. For the BOLD brain region-dependent response results that screened out the specific color temperature that can increase a specific emotion, we calculated those specific color temperature that can make the response effect of a specific emotion reach the maximum response value (that is, the maximum response area value with BOLD).

As shown in step 1510, when the tester has recorded the excitation emotion on the compatible image interaction platform 110 and finishes the lighting program again, the maximum response value is calculated. For example, according to the records in Table 2, the total score of 3,000K (577) is subtracted from the total score of 4,000K (226) to obtain 351. Then, after subtracting the total score (- 105) of 5,700K from the total score (577) of 3,000K, 682 is obtained. The total score of response value under 3,000K lighting after excitation is 1033.

Next, as shown in step 1520 in Fig. 1c, when the tester has recorded the excitation emotion on the compatible image interaction platform 110 and completed the lighting program again, it starts to calculate the maximum response value. For example, according to the records in Table 2, subtract the total score (226) of 4,000K from the total score (577) of 3,000K to obtain - 351. Then, after subtracting the total score (- 105) of 5700K from the total score (266) of 4,000K, 371 is obtained.

Then, as shown in step 1530 in Fig. 1c, after the tester has recorded the excitation emotion induction on the compatible image interaction platform 110 and completed the lighting program, it starts to calculate the maximum response value. For example, according to the records in Table 2, after subtracting the total score (- 105) of 5,700K from the total score (577) of 3,000K, it obtains - 682. Then, the total score (- 105) of 5,700K is subtracted from the total score (- 105) of 4,000K to obtain 371.

According to the above calculation, after the excitation emotion is induced, the excitation emotion can reach the maximum response value at 3,000K lighting. That is, 3,000K illuminance can make excitement get a more obvious additive effect (that is, compared with the total calculated score of 4,000K and 5,700K illuminance, the total calculated score of 3,000K illuminance is 1033, the highest). Therefore, 3,000K illuminance is used as the "effective color temperature" of excitement. For other emotions, such as "effective color temperature" of happiness and amusement, different "effective color temperatures" can be obtained from the above calculation results in steps 1510 to 1530, as shown in Table 5 below.

**Table 5**

| Emotion | Effective Color Temperature |
|---|---|
| Excitement | 3,000K |
| Happiness | 4,000K |
| Amusement | 5,700K |

Next, according to the statistical results in Table 5, the effective color temperature can be regarded as the result of a specific physiological and emotional-dependent response, and this effective color temperature can be regarded as the "enhanced spectrum" of the "blood oxygen-level dependence" of fMRI on a certain emotion. For example, an effective color temperature of 3,000K can represent the "enhanced spectrum" of the fMRI system in "excited" emotions. For example, an effective color temperature of 4,000K can represent the "enhanced spectrum" of the fMRI system in "happy" emotions. For example, the effective color temperature of 5,700K can represent the "enhanced spectrum" of the fMRI system in "amusement" emotions.

Finally, as shown in step 1600, an "enhanced spectrum" database corresponding to the effect of a particular emotion can be established in the fMRI system. The tester is stimulated by the above-mentioned human centric lighting parameters, and the BOLD response of the tester's brain when the tester's brain is stimulated by light is observed and recorded through the fMRI system. The additive and multiplicative response of "blood oxygen-level dependence increase" makes a specific effective color temperature can be regarded as the "enhanced spectrum" of the "blood oxygen-level dependence" of fMRI for a specific emotion. Obviously, the present invention objectively deduces the tester's "enhanced spectrum" under a specific physiological and emotional response based on the statistical result of the BOLD reaching a specific emotional response at a specific "effective color temperature" in Table 5, and This "enhanced spectrum" is used as the evidence of the most synergistic physiological and emotional response to a specific emotion. (Including: excitement, happiness, amusement, anger, disgust, fear, sadness, calm, or neutrality)

It should be emphasized that, in the entire implementation process of FIG. 1b and FIG. 1c, the statistical results in Table 5 are obtained after 100 testers are respectively subjected to a complete test of multiple specific emotions. For example, in terms of excitement, providing an effective color temperature of 3,000K as the "enhanced spectrum" under the excited physiological and emotional response can make the tester's excited emotions produce the most synergistic physiological-emotional response. For example, in terms of happy emotions, providing an effective color temperature of 4,000K as the "enhanced spectrum" under the happy physiological and emotional response can make the tester's happy emotions produce physiological and emotional responses with the strongest synergistic effect. Another example: in terms of amusement emotions, providing an effective color temperature of 5,700K as an "enhanced spectrum" under the amusement physiological and emotional response can make the tester's amusement emotions produce a physiological and emotional response with the strongest synergistic effect.

In addition, it should also be emphasized that the above-mentioned three color temperatures are only representative of the embodiments of the present invention, and not only the lighting of the three color temperatures are used as the "enhanced spectrum" under the three physiological and emotional responses. In fact, the whole process of FIG. 1b and FIG. 1c can be carried out for different emotions (including excitement, happiness, amusement, anger, disgust, fear, sadness, calm or neutral) after 2,000K color temperature is increased by 100K as an interval. Therefore, Table 5 of the present invention is only the result of the disclosure part, not to limit the present invention. The invention is only limited to these three embodiments.

Next, the present invention is to establish an artificial intelligence model of "the correlation between brainwaves and brain images of general physiological and emotional", so that in future commercial promotion, the results of other sensing devices can be directly used to infer physiological and emotional without using fMRI system. Among them, the sensing device matched with the present invention includes electroencephalograph (EEG). In the following embodiments, the electroencephalograph 130 is used to establish the human centric physiological and emotional response to light, and the eye tracker 150 or the expression recognition technology auxiliary program 170 can be used to replace the fMRI system's response to physiological and emotional. However, the eye tracker 150 or the expression recognition technology auxiliary program 170 will not be disclosed in the present invention, but will be announced first.

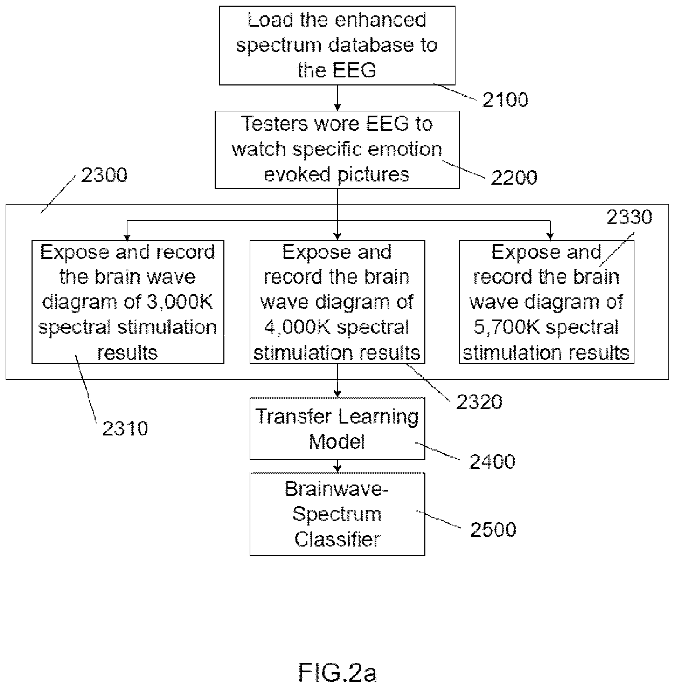

Please refer to FIG. 2a, which is a method for establishing a human centric lighting electroencephalogram response to physiological and emotional according to the present invention. As shown in FIG. 2a, the present invention is a method for establishing the human centric lighting response to physiological and emotional through the electroencephalograph 130, including: first, as shown in step 2100, the "enhanced spectrum" database information in Table 5 is also stored in the memory of the electroencephalograph 130. Next, as shown in step 2200, let the testers wear the EEG, and guide each tester to stimulate various emotions through the elements of known pictures or videos. Afterwards, as shown in step 2300, the intelligent human centric lighting system 100 is activated, and light signal parameters such as spectrum, light intensity, flicker rate, and color temperature that can be changed are provided to stimulate the tester with light. Next, the electroencephalogram file after being illuminated with different color temperatures under a specific emotion is recorded by the electroencephalograph 130. For example, in this embodiment, each tester is first stimulated with excitement, and then, in steps 2310 to 2330, different color temperatures of 3,000K, 4,000K and 5,700K are provided to stimulate the tester respectively, and the tester is recorded in when stimulated by excitement, the electroencephalogram file of the specific emotion after the tester is illuminated with different color temperatures is stored in the device in the memory of the electroencephalograph 130. In the embodiment of the present invention, the electroencephalogram files after 100 testers have completed specific emotional stimulation and lighting have been recorded respectively, therefore, a larger memory is required.

Next, as shown in step 2400, the electroencephalogram files of specific emotions (e.g., excitement, happiness, amusement) stored in the memory of the electroencephalograph 130 is learned through the learning method of artificial intelligence. Since the electroencephalograph 130 can only store the waveform of the electroencephalogram, the electroencephalogram currently stored in the memory of the electroencephalograph 130 is the electroencephalogram file after a known specific triggering emotional stimulus and lighting of different color temperatures. It should be noted that, in the actual test, different testers have different electroencephalogram files for the same emotional stimulus and lighting with the same color temperature. Therefore, during the learning process of step 2400, the present invention needs to classify the electroencephalogram files of specific emotions through the information of the "enhanced spectrum" database. Group the electroencephalogram files with a color temperature of 3,000K, for example: For the electroencephalogram files of happy emotions, only group the electroencephalogram files of different testers with a color temperature of 4,000K. Another example: for the electroencephalogram file of happy mood, only the electroencephalogram files of different testers at 4,000K color temperature are grouped; for example, for the electroencephalogram file of happy mood, only the electroencephalogram files of different testers at 5,700K color temperature are grouped. Then, the EEG is trained through machine learning in artificial intelligence. In the embodiment of the present invention, in particular, a transfer learning model is selected for learning and training.

In the process of learning and training using the transfer learning model in step 2400, the group of electroencephalogram files for specific emotions is learned and trained by counting, calculating and comparing the similarity. For example, when learning and training the group of electroencephalogram files with 3,000K color temperature, it is based on statistics, calculation and comparison of the ranking of the highest similarity and the lowest similarity among the electroencephalogram files with 3,000K color temperature. For example: the ranking with the highest similarity can be regarded as the electroencephalogram file with the strongest emotion, the ranking with the lowest similarity can be regarded as the electroencephalogram file with the weakest emotion, and the electroencephalogram file with the strongest emotion can be regarded as the electroencephalogram file. As the "target value", the electroencephalogram file with the weakest emotion ranking is used as the "starting value". For the convenience of explanation, the most similar at least one electroencephalogram file is taken as the "target value", and the least similar at least one electroencephalogram file is taken as the "start value", and different scores are given, for example: "target value" is given 90 points for similarity, and 30 points for "initial value". Similarly, complete the electroencephalogram files of the series of happy emotions in the 4,000K color temperature category, and the electroencephalogram files of the series of the surprised emotions in the 5,700K color temperature category. Among them, the "start value" and "target value" can be formed into a score interval of similarity.

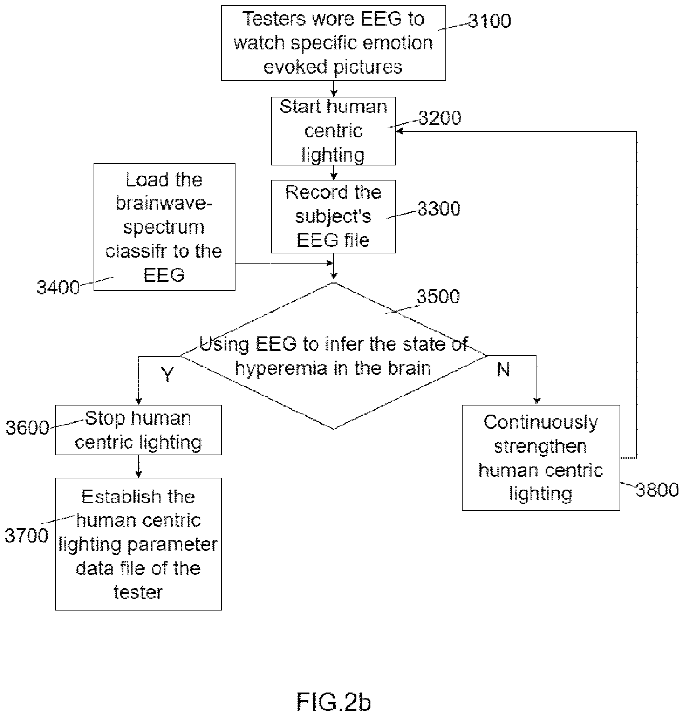

Afterwards, as shown in step 2500, an electroencephalogram classification and grading database in artificial intelligence (it may be referred to as an artificial intelligence electroencephalogram file database) is established. After the step 2400 is passed, the classification results of the "target value" score and the "start value" score are given to the electroencephalogram file groups of various specific color temperatures to form a database, which is stored in the memory of the electroencephalograph 130. The purpose of establishing the electroencephalogram classification and grading database in step 2500 of the present invention is to obtain the electroencephalogram file of the unknown tester after receiving a specific emotional stimulus and giving lighting of a specific color temperature to an unknown tester. After the similarity score interval between the electroencephalogram file of the unknown tester and the electroencephalogram file in the database is compared, it can be used to judge or infer the current state of the unknown tester's hyperemia reaction in the brain. The detailed process is shown in FIG. 2b shown.

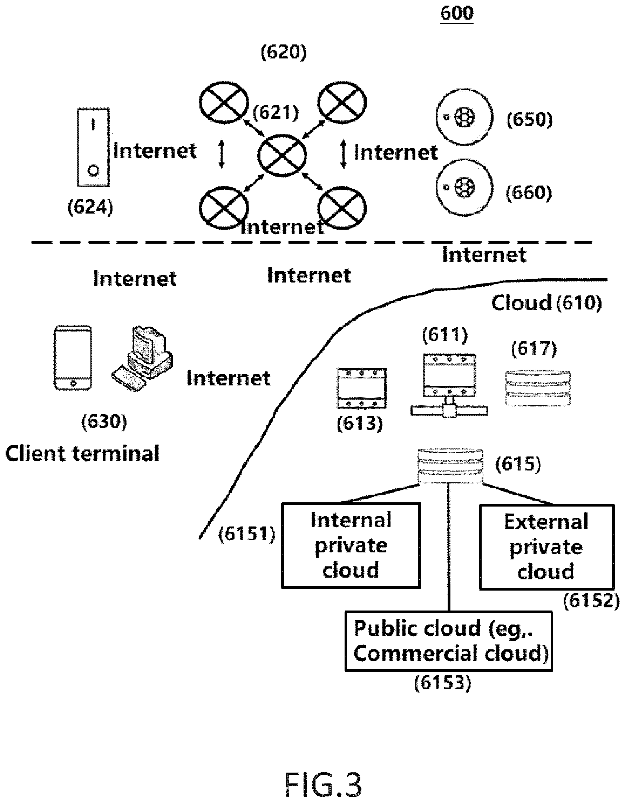

Next, please refer to FIG. 2b, which is a lighting database for constructing a user's effective human centric lighting according to the present invention. First, as shown in step 3100, the tester is put on the electroencephalograph 130, and the tester is allowed to watch a picture evoked by a specific emotion. Afterwards, as shown in step 3200, the intelligent human centric lighting system 100 is activated by management control module 611 (as shown in FIG. 3) in an environment (e.g., a test space) that has been configured with various adjustable multispectral lighting modules. To provide light parameters such as spectrum, light intensity, flashing rate, color temperature and other light signal light parameters that can be changed. Next, as shown in step 3300, obtain the electroencephalogram file of the tester after the induced emotion and lighting, and store it in the memory module 617 of the cloud 610 (as shown in FIG. 3). Next, as shown in step 3400, import the artificial intelligence electroencephalogram file database into the management control module 611. Wherein, the management control module 611 will set a score of whether the similarity is sufficient. For example, when the similarity score is set to be more than 75 points, it means that the tester's brain hyperemia reaction is sufficient. Next, as shown in step 3500, in the management control module 611, the similarity between the tester's electroencephalogram file and the artificial intelligence electroencephalogram file is compared. For example: when the similarity score of the tester's electroencephalogram files after comparison is 90 points, the management control module 611 immediately judges that the tester's brain hyperemia reaction is very sufficient, so it will go to step 3600 to terminate the person with a specific emotion Due to lighting test. Next, go to step 3700, record the human centric lighting parameters in the tester's brain when the hyperemia response has reached the stimulus, into a database and store in the memory module 617.

Next, in the procedure of step 3500 in FIG. 2b, if the similarity score after the comparison between the tester's electroencephalogram file and the artificial intelligence electroencephalogram file is 35 points, the management control module 611 will determine the tester's brain If the hyperemia reaction is insufficient, step 3800 will be performed, and the management control module 611 will continue to strengthen the human centric lighting test, including: according to the similarity score, the management control module 611 can control it to provide an appropriate increase in the lighting time or increase light intensity. Then, the electroencephalogram file after increasing the lighting time or intensity is obtained again through step 3300. After step 3400, the human centric lighting test is not stopped until the similarity score reaches the set similarity score of more than 75 points. Wherein, when the management control module 611 determines that the hyperemia reaction in the tester's brain has reached the stimulation, in step 3700, a data file of the human centric lighting parameters of the tester is created. Finally, the management control module 611 will form a "human centric lighting parameter database" of the human centric lighting parameters of each tester and store it in the memory module 617. Obviously, when there are more testers, the artificial intelligence electroencephalogram file database of the present invention will learn more electroencephalogram files, so that the similarity score of the present invention is more and more accurate.

After the artificial intelligence model of the "human centric lighting parameter database" is established, after the intelligent human centric lighting system 100 is activated, the present invention can deduce the result only by observing the electroencephalogram file of the electroencephalograph 130, that is, it can be deduced The physiological and emotional changes in the brain images of the new test subjects can be inferred based on the artificial intelligence model of the "Human Centric Lighting Parameter Database" without using a high-value fMRI system. So that the intelligent human centric lighting system 100 can be promoted and used commercially. In addition, in order to enable the "human centric lighting parameter database" to be used commercially, the "human centric lighting parameter database" may be further stored in the internal private cloud 6151 in the cloud 610.

Next, please refer to FIG. 3, which is a system architecture diagram of the intelligent human centric lighting system 100 of the present invention. As shown in FIG. 3, the overall architecture of the intelligent human centric lighting system 100 of the present invention can be divided into three blocks, including: a cloud 610, a lighting field end 620 and a client terminal 630. The internet is used as a connection channel, so the three blocks can be distributed in different areas, and of course they can be configured together. The cloud 610 further includes: a management control module 611, which is used for cloud computing, cloud environment construction, cloud management or use of cloud computing resources, etc., and also allows users to access, construct or modify the content in each module through the management control module 611. The consumption module 613 is connected with the management control module 611 and is used as a cloud service subscribed and consumed by the user. Therefore, the consumption module 613 can access various modules in the cloud 610. The cloud environment module 615, connected with the management control module 611 and the consumption module 613, divides the cloud background environment into an internal private cloud 6151, an external private cloud 6153, and a public cloud 6155 (for example, a commercial cloud), etc., and can provide system providers or an interface to a user's external or internal service. The memory module 617 is connected to the management control module 611 and is used as a storage area of the cloud background. The technical contents required to be executed by each module in the present invention will be described in detail in the subsequent different embodiments. The lighting field end 620 can communicate with the cloud 610 or the client terminal 630 through the internet. The lighting field end 620 is provided with a LED lamps group 621 composed of a plurality of light-emitting devices. And the client terminal 630 can communicate with the cloud 610 or the lighting field end 620 through the internet. Among them, the client terminal 630 of the present invention includes general users and editors who use the intelligent human centric lighting system 100 of the present invention for various commercial operations, all of which belong to the client terminal 630 of the present invention, and the representative device of the client terminal 630 or the device can be a fixed device with computing function or a portable intelligent communication device. In the following description, the user, creator, editor or portable communication device can all represent the client terminal 630. In addition, in the present invention, the above-mentioned internet may be an Artificial Intelligence of Things (AIoT).

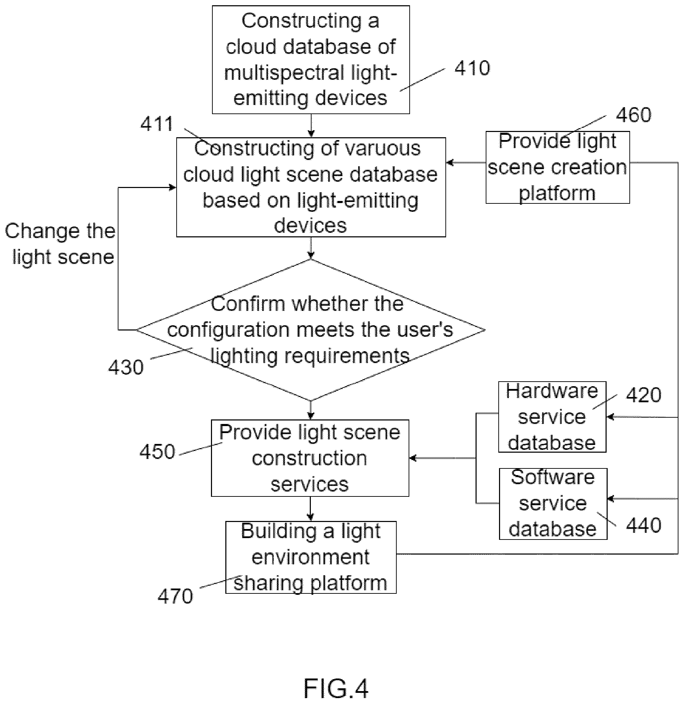

Please refer to FIG. 4, which is a method for constructing light environment platform 400 according to the present invention. In the process of FIG. 4, the process is performed in the system of the intelligent human centric lighting system 100 in FIG. 3, wherein the intelligent human centric lighting system 100 has a plurality of LED lamps group 621 providing different spectrums. In particular, the present invention should illustrate that the LED lamps group can selectively modulate the voltage or current of the individual LED lighting to combine a multispectral combination with a specific color temperature, so as to provide a multispectral combination for lighting. Of course, the present invention can also choose to use a plurality of LED lamps with a specific luminous spectrum, by providing a fixed voltage or current to make each LED lamps that is energized emit its specific spectrum to combine a multispectral combination with a specific color temperature come to light. In addition, it should be emphasized that the LED lamps group 621 with different spectrums in the present invention need to emit different spectrums through a plurality of LED lamps group 621 according to the actual lighting process. Therefore, the LED lamps group 621 with different spectrums can be composed of different. The spectrum of light-emitting devices can also be composed of multiple identical light-emitting devices, and the spectrum emitted by the light-emitting devices can be modulated into different spectrums by controlling the power provided by the device. Therefore, the present invention does not limit the embodiments of the above LED lamps group 621 with different spectrums, and of course also includes light-emitting devices with different spectrums formed by other means.

First, as shown in step 410, a multispectral light-emitting device cloud database is constructed. The "human centric lighting parameter database" stored in the memory module 617 or stored in the internal private cloud 6151 is loaded into the management control module 611 by the management control module 611 in the cloud 610. Among them, the "human centric lighting parameter database" stored in the cloud 610 already knows that various color temperatures can make people (or users) get corresponding emotional stimulation. For example: a specific color temperature can make a specific emotion have a stimulating effect of a multiplicative response.

Next, as shown in step 411, a cloud database of light scenes of the multispectral lighting device is to be constructed. Since each color temperature can be combined by different multiple spectrums, that is to say, the same color temperature can be combined by different multiple spectrums. For example, when we want to build a spectrum that can provide 4,000K color temperature, we can choose the multispectral combination of Maldives at 4,000K color temperature, or we can choose the multispectral combination of 4,000K color temperature in Bali, Indonesia, or further choose Monaco Beach at 4,000K multispectral combination of color temperature. Obviously, these multispectral combinations with a color temperature of 4,000K have different multispectral combinations according to their geographical location (including latitude and longitude), time/brightness/flashing rate and other factors. Therefore, in step 411, according to the relationship between the color temperature and the corresponding emotions in the "human centric lighting parameter database", the cloud 610 can collect different multispectral combinations of specific color temperatures in different geographical locations on the earth, for example, collecting on the earth different multispectral combinations at 4,000K color temperature in different geographic locations (e.g., 4,000K color temperature at different latitude and longitude), or collect different multispectral combinations at 4,000K color temperature at different times on earth (e.g., 4,000K color temperature at 9:00 am). Afterwards, the collected different multispectral combinations of different geographic locations on the earth at specific color temperatures are also stored in the memory module 617. Through the control of the management control module 611 in the intelligent human centric lighting system 100, the parameters of various multispectral combinations with specific color temperature (i.e., time at different geographical locations or locations) can be adjusted by controlling a plurality of LED lamps group 621 with different spectra, which are stored in the memory module 617. The intelligent human centric lighting system 100 of the present invention can construct a "cloud database of multispectral light-emitting devices" with multispectral combinations of various color temperatures formed in different geographical locations. Obviously, the "multispectral light-emitting device cloud database" constructed in step 411 is the result of adjustment by multiple LED lamps group 621 with different spectra. In addition, it should be emphasized that after mastering the stimulation effect that different specific color temperatures can make specific emotions have additive reaction, when forming the multispectral combination of various color temperatures, in addition to the multispectral combination according to different geographical locations on the earth, the present invention can also consider different longitude and latitude/time/brightness/flicker rate and other factors through artificial intelligence. The invention does not limit the synthesis or combination of multispectral combinations different from the actual geographical location. Obviously, in step 411, the present invention has constructed a light scene database with multispectral light-emitting device cloud database. Therefore, according to the constructed database of different light scenes, the intelligent human centric lighting system 100 can provide multispectral lighting services under different color temperatures through the multispectral lighting device. At this time, the "multispectral light-emitting device cloud database" constructed in step 411 can be stored in the memory module 617 or in the internal private cloud 6151 or in the public cloud 6155 (e.g., commercial cloud).

As shown in step 430, it is determined whether the lighting of the multispectral combination is effective. Since different users may have different effects on emotional stimulation or influence through the spectrum of human centric lighting, it is necessary to adjust the lighting spectrum of the multispectral combination. Next, when the client terminal 630 enters the intelligent human centric lighting system 100 of the present invention, he can go through the management control module 611 to the memory module 617 or the "multispectral light-emitting device cloud database" in the internal private cloud 6151 or the public cloud 6155, select the emotion you want to achieve. After that, the management control module 611 can go to the memory module 617 or the "multispectral light-emitting device cloud database" in the internal private cloud 6151 or the public cloud 6155, select a default multispectral combination, and control multiple different spectrums. The LED lamps group 621 is used to adjust the multispectral combination of the desired mood, and let the adjusted multispectral combination perform a lighting program for the user. For example, when the user wants to adjust the mood to happiness or amusement, according to the "human centric lighting parameter database", the user can choose to use the color temperature of 4,000K to illuminate. At this time, the user can go to the default multispectral combination provided by the "multispectral light-emitting device cloud database", or the user can choose a multispectral combination that can provide the color temperature of 4,000K, of course, the user can also choose a specific multispectral combination used by the most people for irradiation. At this time, when the user uses the multispectral combination provided by the intelligent human centric lighting system 100 in the "multispectral light-emitting device cloud database", the present invention is called the default multispectral combination.

Next, as shown in step 430, when the user selects a multispectral combination preset as happiness or amusement (for example, the system defaults to the multispectral combination of Maldives at 4,000K color temperature), and carries out the lighting program through the multispectral combination adjusted by a plurality of LED lamps group 621 with different spectra, for example, after 15 minutes of lighting program, the user can judge or evaluate whether the multispectral combination of irradiation has effectively achieved happiness or pleasant emotion. Among them, the way for users to judge or evaluate whether they have effectively achieved happiness or amusement emotions can be determined according to their own feelings.

Continuing as shown in step 430, when the user intuitively feels that he or she has effectively achieved a happy or amusement mood, the user experience can be recorded and stored in the external private cloud 6153 or the public cloud 6155, for example, after the record of use experience is stored in the external private cloud 6153, it can be used as the user's own use experience file. For example, of course, the use experience can also be recorded and stored in the public cloud 6155 to provide reference for other users. If the user does not feel happy or amusement after 15 minutes of lighting, the user can adjust the multispectral combined spectral recipe through the management control module 611, for example, adjust the lighting time to 30 minutes, or replace for a multispectral combination light scene, for example, go back to step 411 to reselect another multispectral combination light scene, and replace the multispectral combination light scene from the default Maldives to Bali, Indonesia. After that, in step 430, the multispectral lighting procedure is performed again for 15 minutes, and after the lighting procedure is completed, it is judged or evaluated again whether the lighting of Bali's multispectral combination has effectively achieved a happy or amusement. Until the user feels that after the multispectral lighting of the current light scene, the happy or amusement mood has been effectively achieved, the effective light scene use experience can be recorded and stored to form a "spectral recipe", and finally, and store this "spectral recipe" in the external private cloud 6153 or the public cloud 6155 to form a shared platform. Obviously, there are multiple "spectral recipe" stored in the shared platform. The contents stored in the shared platform include: the lighting control parameters of the light scene in which the user achieves the specific emotional effect and the multispectral combination of the LED lamps group 621 with multiple different spectra generating the specific emotional light scene (hereinafter referred to as the lighting parameters).

Finally, as shown in step 470, a light environment platform is constructed. After the user records and stores the effective use experience in the external private cloud 6153 or public cloud 6155, in addition to being the user's own use experience file, the intelligent human centric lighting system 100 can record and store the effective experience data of many users in the internal private cloud 6151 or public cloud 6155 after massive data analysis in step 470, the information stored in the public cloud 6155 forms a "sharing platform" of the optical environment. For example, the intelligent human centric lighting system 100 can analyze the massive data of many users' experience data, and then can obtain the ranking of the "spectral recipe" of different light scenes with specific color temperature selected by the user. This ranking can provide a reference for the user to select the "spectral recipe". Further, after analyzing these massive data, we can get different indicators to provide users with reference. For example, in terms of different multispectral combinations of 4,000K color temperature, different usage orders can be made according to the user's gender, age, race, season, time, etc., so that the light environment platform constructed by the present invention can be used as other designers who are interested or willing to provide human centric spectral recipe, use these indicators for commercial services and operations. Therefore, the light environment platform constructed by the present invention can allow the intelligent human centric lighting system 100 to provide various usage experiences after curation or algorithm operation as the default multispectral combination. In addition, users can also choose a "spectral recipe" that is most selected by the user for lighting according to the user experience data after calculation.

In the embodiment of FIG. 4, step 450 can exist selectively. For example, if the user only establishes the database for user's own use, user can directly record and store the effective use experience in the external private cloud 6153 or public cloud 6155 after determining that it is valid in step 430 without this step 450. The database of the "spectral recipe" construction service to be constructed in step 450 will be described in the following embodiments.

Next, in FIG. 4, there is another embodiment that can be used for commercial services and operations, it can provide an authoring platform that allows different users or creators to create new "spectral recipe" through this authoring platform. As shown in step 460, a "spectral recipe" authoring platform is provided in the intelligent human centric lighting system 100. The Client terminal 630 using this "spectral recipe" authoring platform can be distributed all over the world and can communicate with each other through the internet. The "light environment platform" connection in the cloud 610 of the present invention. Obviously, the present invention defines a "spectral recipe" creation platform here, that is, a "spectral recipe" creation platform means that a user or client terminal 630 can connect to the public cloud 6155 in the cloud 610 or a "sharing platform" through the internet, so that users or clients terminal 630 can use the "spectral recipe" information on the public cloud 6155 or "sharing platform" for editing work. Therefore, after the user or client terminal 630 obtains various calculated usage experience data from the "sharing platform", the user or client terminal 630 can obtain the experience data based on the user's gender, age, race, season, time, etc., edit or create by client terminal 630 to construct an edited "spectral recipe" of multi-scene or multi-emotional multispectral combination.

Firstly, the first embodiment of the present invention uses the "spectral recipe" creation platform for commercial service and operation. For example, for the emotional stimulation result of 4,000K color temperature, the user or client terminal 630 can combine different light scenes through the "spectral recipe" creation platform. For example, the first paragraph of client terminal 630 selects the multi spectra combination of Maldives, the second paragraph is to choose the multispectral combination of Bali, and finally, the third paragraph ends with the multispectral combination on the beach of Monaco. In this way, after each segment is matched or configured with the spectral irradiation time, a new spectral combination of the multi-light scene can be formed. Finally, the spectral combination of the multi-light scene can be stored in the "sharing platform", which can provide other users with options. The spectral combination of this multi-light scene serves as the "spectral recipe" for its emotional adjustment. Among them, the creation platform of the present invention can also select a specific color temperature at a specific time, for example, when a user wants to perform emotional stimulation at 9:00 am, the user can further select the aforementioned three multispectral combinations at 9:00 am. Further, the multispectral irradiation time of each segment can be configured to be the same or different, for example, each segment is irradiated for 15 minutes. The first stage and the third stage may be arranged for 15 minutes, and the second stage may be arranged and irradiated for 30 minutes, and these same or different time configurations can be selected by the user.

Secondly, in the second embodiment of the present invention using the "spectral recipe" creation platform, different color temperatures (that is, multiple emotions) can be combined to achieve specific emotional stimulation results through lighting with multiple emotions, for example, the client terminal 630 can use the "spectral recipe" creation platform to combine multiple emotions. For example: the first paragraph of the client terminal 630 is to select a multispectral combination of 4,000K (happy emotion), and the second paragraph is to select 3,000K (excited emotion). Finally, the third paragraph ends with a multispectral combination of 5,700K (amusement emotion). In this way, after each segment is matched or configured with multispectral irradiation time, a new multi-emotional multispectral combination can be formed. Finally, this multi-emotional multispectral combination can be stored in the "sharing platform", which can provide users with choices. This multispectral combination of multiple emotions serves as the "spectral recipe" for its emotional adjustment. Similarly, the creation platform in this embodiment can also select a specific color temperature at a specific time. For example, when the user wants to perform emotional stimulation at 9:00 am, user can further select the above three paragraphs spectral combination at 9:00 am. Further, the multispectral irradiation time of each segment can be configured to be the same or different, for example, each segment is irradiated for 15 minutes. The first stage and the third stage may be arranged for 15 minutes, and the second stage may be arranged and irradiated for 30 minutes, and these same or different time configurations can be selected by the user.

Next, it should be further explained that after the editing or creation of the above-mentioned first embodiment (multispectral combination of multi-light scenes) and the second embodiment (multispectral combination of multi-mood light scenes) is completed, step 430 also needs to go through step 430. Process to step 470. For example, step 411 should be passed first, and the lighting should be performed through the multi-spectrum adjusted by a plurality of LED lamps group 621 with different spectra. Next, after step 430, it is determined whether the combination of the above-mentioned "spectral recipe" achieves the effect that the creator wants, including: the spectral combination of the multi-light scene of the first embodiment and the irradiation time of the corresponding configuration, and the second embodiment the multi-emotional spectral combinations and configured exposure times. If the effect set by the creator can be achieved, the control parameters of the LED lamps group 621 required to construct these "spectral recipe" can be formed into a database that can provide a "spectral recipe" construction service, as shown in step 450. Then, as shown in step 470, after uploading the database of the "spectral recipe" construction service constructed in step 450 to the cloud, these "spectral recipe" can be added to the public cloud 6155 of the present invention to form a light environment platform. If after step 430, it is determined that a certain "spectral recipe" cannot achieve the effect set by the creator, then you can go back to step 411 to adjust the irradiation time of each segment, or change to a different light scene combination (the first embodiment) or change different emotional combinations (the second embodiment), until these "spectral recipe" can achieve the effect set by the creator, then the process from step 430 to step 470 can be used to judge that these are effective. The "spectral recipe" of the light environment is added to the public cloud 6155 of the present invention to form a light environment platform.

In addition, in order to make the multi-scene combination or multi-emotion combination of the "spectral recipe" creation platform effective, it may be necessary to go through a specific LED lamps group 621 or a specific configuration diagram to achieve the best effect. Therefore, the present invention is further configured with a database of hardware services, as shown in step 420, and further configured with a database of software services, as shown in step 440. Among them, the database of hardware services and the database of software services are also embedded with some management information when building "spectral recipe", including installation or delivery of hardware devices, or including providing and building these "spectral recipe" through the database of software services software services such as space design, scene planning, or interface setting required by spectral recipe. In addition, the database of the hardware service and the database of the software service may be configured at one end of the client terminal 630, or may be configured in the cloud 610, which is not limited in the present invention. Obviously, in the embodiment of FIG. 4, the present invention has provided the process of constructing various "spectral recipe" and the database of "spectral recipe" construction services, as well as the hardware services required for the construction of "spectral recipe". The database and software service database have been established in the light environment platform. It should be noted that the "sharing platform" finally established in step 470 is a "spectral recipe" that can provide effective spectral combinations of multi-light scenes and multi-emotional light scenes. "Sharing Platform" provides various commercial services and operations.

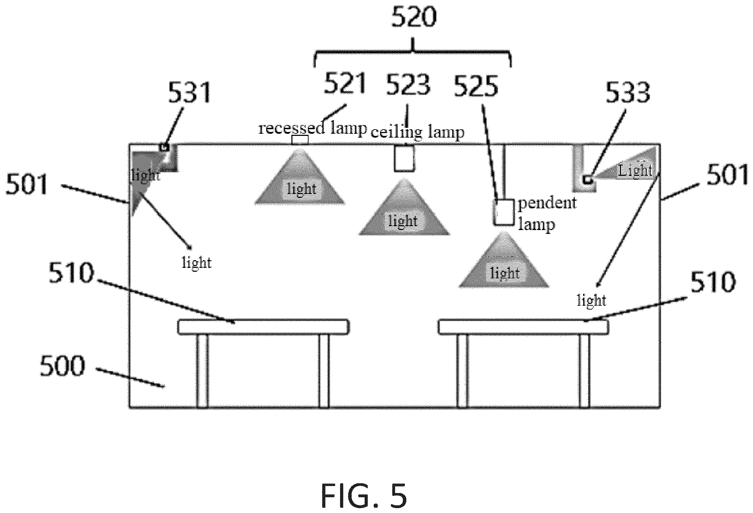

Please refer to FIG. 5, which is a schematic diagram of the circadian rhythm lighting scenario formed by the lamp group in a space of the present invention. As shown in FIG. 5, a space 500 of the present invention is provided with a plurality of lamps, including a recessed lamp 521 embedded in the ceiling, a ceiling lamp 523 protruding from the ceiling, and a pendant lamp 525 hanging from the ceiling. The light emitted by the lamps in these three lamps directly irradiates the light to the surface of the object (e.g., desktop 510). These lamp groups are called direct (or horizontal) lighting lamp groups 520 in the present invention. In addition, the invention also configures a plurality of other lamps in the same space 500, and the light emitted by these lamp groups first irradiates the wall 501, and then the wall 501 reflects or scatters the light to the surface 510 of the object. The invention calls these lamp groups indirect (or vertical) lighting lamp groups 530, wherein, the indirect (or vertical) lighting fixture group 530 includes a ceiling lamp 531 that shines light on the wall 501 at an angle, and a chandelier or track lighting 533 that shines light on the wall 501 at an angle. Obviously, in the space 500 of the present invention, a plurality of direct (or horizontal) lighting lamp groups 520 and a plurality of indirect (or vertical) lighting lamp groups 530 are configured, and the proportion of the light emitted by these lighting lamp groups irradiating the surface 510 of the object is controlled as the lighting scenario of the present invention. By controlling the light proportion of these lighting fixture groups, the lighting light can be provided directly through the light sensor (such as the ambient light sensor 650) according to the scenario proportion. In addition, the invention can also control the light sensor 650 through the controller 611 to provide the lighting light according to the scenario proportion provided by the controller.

Next, please refer to Table 6, which is an embodiment of the circadian rhythm lighting scenario database for indoor lighting provided by the present invention. As shown in Table 6 below, the circadian rhythm lighting scenario database is a preset specific time interval based on personal work and rest habits or work cycles under the daily 24-hour daylight demand cycle. From this time interval, the circadian rhythm light parameters of indoor lighting in the process of corresponding circadian rhythm changes or daylight changes can be mapped, including equivalent black lux (EML) Circadian action factor (CAF), sunlight illuminance (Lux) and color temperature change, so that users can quickly select the indoor circadian rhythm lighting they need. Among them, the above Equivalent Melanopic Lux (EML) is mainly calculated by multiplying the vertical visual illuminance in a space by the ratio of lighting sources. Therefore, EML value represents the level of a kind of indoor light stimulation to human physiological cycle, and is used to quantify the biological effect of light on human beings. Therefore, different daily activities are suitable for different EML values. For example, high EML values are required during the day (08:00-12:00) or when concentration is required. In contrast, low EML values are required at night (22:00-07:00) or when relaxation is required. In addition, the above-mentioned Circadian Action Factor (CAF) is mainly calculated according to the lighting of the lamp group, and is used to measure the stimulation of the lighting of the lamp group to the human body. For example, when the Circadian Action Factor (CAF) value is higher, the higher the stimulation of the light to the human physiology, which can refresh people. The lower the CAF value, the lower the stimulation of light on the human body, which makes people feel relaxed. Obviously, the difference between CAF and EML is that the former measures the stimulation of the lighting spectrum of the lamp group to the human body, while the latter measures the stimulation of indoor light to the human physiological cycle. But they are the same: the higher the value, the higher the stimulation to the human body, which can boost the spirit. The lower the value, the lower the stimulation to the human body and help relax the body and mind.

**Table 6**

| Time division | | 22:00-07:00 | 07:00-08:00 | 08:00-12:00 | 12:00-13:30 |
|---|---|---|---|---|---|
| Circadian rhythm | | Deep sleep | morning rise | Morning | Lunch break |
| EML (lux) | | <50 | 150 | 200 | 100 |
| CAF | | 0.3 | 0.57 | 0.86 | 0.47 |
| Illuminance (lux) | | 100 | 250 | >500 | 200 |
| CT(K) | | <3000 | 4000 | 5700 | 3500 |
| Scenario | Direct (%) | 0-20 | 20∼40 | 80∼100 | 40∼60 |
| | Indirect (%) | 80∼100 | 60∼80 | 0∼20 | 40∼60 |
| Cortisol index value | | 2.9∼17.3 | 3.7∼19.4 | 3.7∼19.4 | 3.7∼19.4 |

| Time division | | 13:30-17:00 | 17:00-21:00 | 21:00-22:00 |
|---|---|---|---|---|
| Circadian rhythm | | Afternoon | Evening | Bedtime |
| EML (lux) | | 200 | 150 | 75 |
| CAF | | 0.57 | 0.47 | 0.36 |
| Illuminance (lux) | | >500 | 300 | 150 |
| CT (K) | | 4000 | 3500 | 3000 |
| Scenario | Direct (%) | 60∼80 | 40∼60 | 20∼40 |
| | Indirect (%) | 20∼40 | 40∼60 | 60∼80 |
| Cortisol index value | | 3.7∼19.4 | 2.9∼17.3 | 2.9∼17.3 |

According to the above, in the embodiment of the circadian rhythm lighting scenario database of the indoor lighting of the invention, as shown in Table 6, according to the circadian rhythm of the human body, the daily 24 hours are divided into seven sections: deep sleep (22:00-07:00), morning rise (07:00-08:00), morning (08:00-12:00), lunch break (12:00-13:30), afternoon (13:30-17:00), evening (17:00-21:00) and bedtime (21:00-22:00), the corresponding indoor circadian rhythm lighting parameters of these sections are formulated to form the circadian rhythm lighting scenario database of indoor lighting. The EML, CAF and illuminance (Lux) in Table 6 refer to the threshold value of indoor circadian rhythm lighting parameters (that is, the minimum value to be reached). For example, the circadian rhythm at 22:00-07:00 is a deep sleep time. Therefore, the lower the indoor circadian rhythm lighting parameters during this period, the better. Therefore, the EML setting in the database should be less than 50, the CAF setting should be 0.3, the lighting (Lux) setting should be 100, and the color temperature (K) setting should be less than 3000. For example, the time difference is in the morning (08:00-12:00). Because the sunlight is gradually increasing, and it is also the time when human activity and alertness are the highest, the EML setting in the database should be greater than 200, CAF setting should reach 0.86, lux setting should be greater than 500, and color temperature (K) setting should be greater than 5700.

In addition, in order to enable the circadian rhythm of the human body to feel the real lighting experience, the invention particularly regulates the lighting light of the indoor lamp group in scenario, and sets the circadian rhythm scenario parameters. As shown in the scenario column in Table 6, for example, the circadian rhythm at 22:00-07:00 is a deep sleep time. Therefore, the circadian rhythm scenario parameter setting of indoor light at this time is mainly provided by the indirect (or vertical) lighting lamp group 530. In the preferred embodiment of the invention, the circadian rhythm scenario parameter setting at this time is that the indirect (or vertical) lighting lamp group 530 provides 80-100% of the light, the direct (or horizontal) lighting fixture group 520 provides 0-20% of the light. For example, in the morning (08:00-12:00), due to the gradual enhancement of sunlight, the indoor light at this time is mainly provided by the indirect (or vertical) lighting group 530. In the preferred embodiment of the invention, the circadian rhythm scenario parameter setting at this time is that the direct (or horizontal) lighting group 520 provides 80-100% of the light, while the indirect (or vertical) lighting group 530 provides 0-20% of the light. For another example, the time difference is at evening (17:00-21:00). Since the sun has already sunk, the ambient light is uniform or soft. At this time, the indoor light is mainly provided by the direct (or horizontal) lighting fixture group 520 and the indirect (or vertical) lighting fixture group 530. Therefore, the circadian rhythm scenario parameter setting at this time is that both lighting fixture groups provide 40-60% of the light. The above direct (or horizontal) lighting fixture group 520 and indirect (or vertical) lighting fixture group 530 are shown in FIG. 5.

According to the above, in addition to the standard time division specified in Table 6, the invention can also customize the time division according to the needs of users, as shown in Table 7 below. For example, the specific time interval given according to the user's needs is as follows:
(1) Lighting scenario setting of three shift system and eight hour work
(2) 06:30~08:30 lighting scenario setting of fresh morning time
(3) 13:30-17:30 setting of lighting scenario in leisure afternoon
(4) 20:00~23:00 lighting scenario setting for relaxing sleep time

Obviously, the invention can give different time differentiation and corresponding circadian rhythm parameter setting according to the user's needs or preferences. The specific operation example of the three shift system in Item 1 above is described as follows. For example, if the user wants to set the lighting scenario setting for the three shift 8-hour work, it means that the three shift 8-hour work period (including: 8:00 a.m. to 5:00 p.m. in the normal shift, 4:00 p.m. to 1:00 a.m. in the afternoon shift, 12:00 midnight in the night shift to 9:00 a.m. in the next day) is expected to control the physiological state when it is "suitable for daytime work environment that inhibits melatonin secretion and makes people feel awake and excited". Then, the work field can be in the same lighting scenario during the three shift 8-hour work period.

**Table7**

| **Time division** | | **08:00-17:0 0** | **06:30-08:30** | **13:30-17:3 0** | **20:00-23:0 0** |
|---|---|---|---|---|---|
| **Circadian rhythm** | | **Three-shift system** | **Morning rise** | **Afternoon** | **Sleep** |
| **EML (lux)** | | **200** | **150** | **200** | **<50** |
| **CAF** | | **0.86** | **0.57** | **0.57** | **0.3** |
| **Illuminance (lux)** | | **>500** | **250** | **>500** | **100** |
| **CT (K)** | | **5700** | **4000** | **4000** | **<3000** |
| **Scenario** | **Direct (%)** | **0∼20** | **60∼80** | **0∼20** | **40∼60** |
| | **Indirect (%)** | **80∼100** | **20∼40** | **80∼100** | **40∼60** |
| **Cortisol index value** | | **3.7∼19.4** | **3.7∼19.4** | **3.7∼19.4** | **2.9∼17.3** |

In particular, in the embodiment of the invention, the circadian rhythm lighting parameters (including EML, CAF, sunlight illuminance, color temperature, etc.) set in the indoor lighting circadian rhythm lighting scenario database in tables 6 and 7 are formed by the direct (or horizontal) lighting lamp group 520 and the indirect (or vertical) lighting lamp group 530 according to the time division. The light scenario of indoor lighting is provided by the circadian rhythm scenario parameters of the invention, so that the indoor lighting system constructed in the invention can provide a more comfortable and realistic circadian rhythm lighting effect. In the application of the following embodiments, the circadian rhythm lighting parameters and circadian rhythm scenario parameters in tables 6 and 7 will be collectively referred to as circadian rhythm parameters.

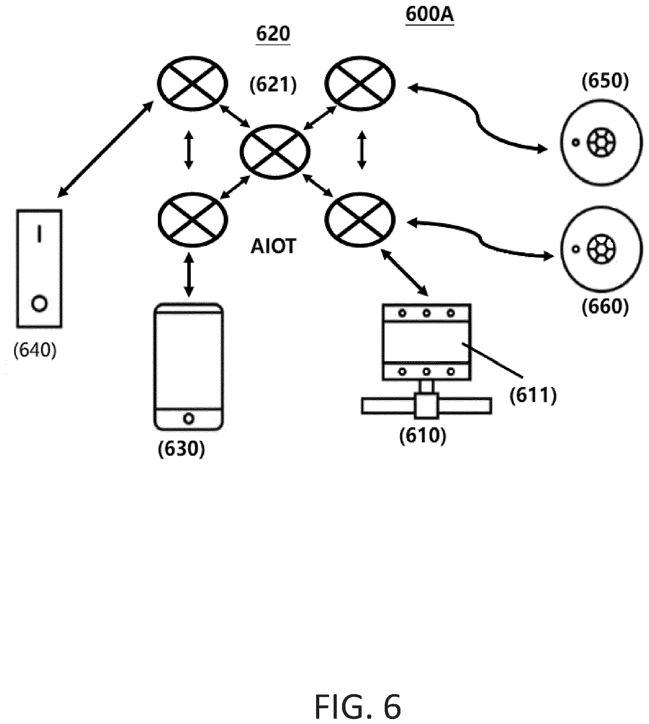

The invention further provides a lighting system, as shown in FIG. 6. This lighting system can be used to establish a multispectral circadian rhythm lighting database, or it can be a lighting system with the ability to provide multispectral circadian rhythm lighting scenario information. The lighting system 600 of the invention comprises a cloud terminal 610, a management control module 611, a lighting field end 620 of intelligent lighting, and a portable communication device 630. The device configured in the lighting field end 620 includes a lamp group or light-emitting device 621, at least one occupancy sensor 660, at least one ambient light sensor 650, and a switching device 640. The lamp group 621 configured in the lighting field end 620 is the same as the plurality of direct lighting lamp groups 520 and the plurality of indirect lighting lamp groups 530 configured in the space 500 (FIG. 5). In addition, the client can use the portable communication device 630 to download the "multispectral lighting device cloud database" from the Internet to the cloud 610, and start the lamp group 621 through the portable communication device 630 to establish a multispectral circadian rhythm lighting scenario database, so as to form a lighting system with multispectral circadian rhythm lighting scenario. In this embodiment, the client may be in the remote end or in the space 500 at the near end. When the client is at the remote end, the Internet is an intelligent Internet of things (AIoT) formed by the Internet of things (IoT) and artificial intelligence (AI). When the client is at the near end, the Internet is a wireless communication protocol formed by a gateway, including Wi-Fi, ZeeBee or Bluetooth. In the embodiment of the invention, the lighting field end 620 or the space 500 of the light-emitting device can be a conference room, a classroom, an office, a social place or a factory, and can provide multiple users with the lighting of the circadian rhythm lighting scenario in the intelligent lighting field.

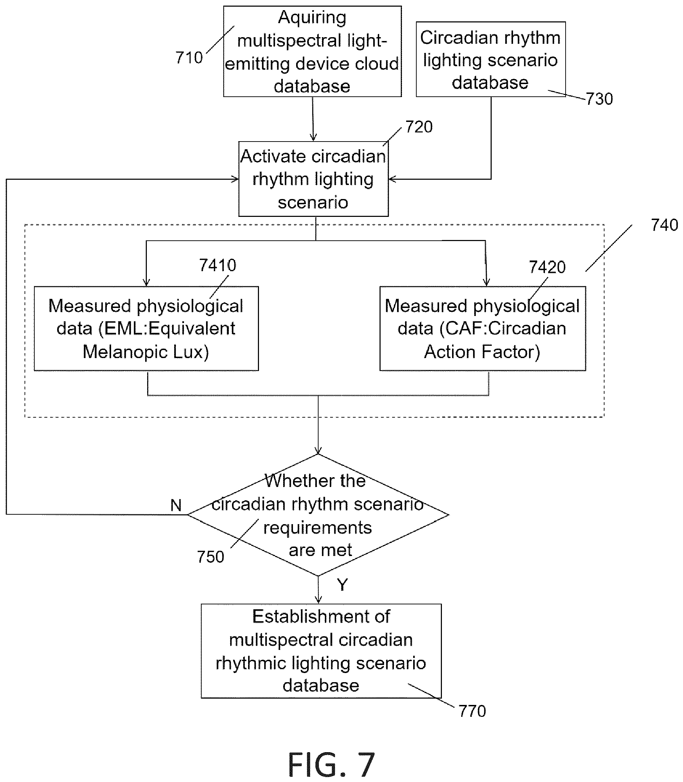

Next, referring to FIG. 7, there is a method for establishing a multispectral circadian rhythm lighting database of the present invention. First, as shown in step 710, the "multispectral light-emitting device cloud database" can be retrieved from the portable communication device 630 to the cloud 610 through the network (e.g., AIoT). The "multispectral light-emitting device cloud database" can be the same as the database formed in step 411. As a result of step 411, a light scene database with a plurality of multispectral light-emitting devices has been constructed. Therefore, after the lighting system 600 has obtained the light scene data of the multispectral light-emitting device in step 710, the lighting system 600 can provide the lighting service of different color temperatures by driving the lamp group 621 (or the multispectral light-emitting device) through the multispectral lighting parameters of the selected specific color temperature. Among them, the multispectral lighting parameters of the lamp group 621 include: spectrum, light intensity, flicker frequency, color rendering (RA), etc.

Next, as shown in step 720, "start circadian rhythm lighting scenario lighting". In the process of "starting circadian rhythm lighting scenario lighting", first, the lamp group 621 in the lighting system 600 has provided a multispectral lighting service of color temperature of geographical location through the portable communication device. Next, the application program (APP) in the portable communication device 630 starts or calls the circadian rhythm lighting scenario database of Table 6, as shown in step 730. At this time, the lamp group 621 will distinguish the set circadian rhythm parameters according to the actual time, so that the lighting at the lighting field end 620 reaches the set values of Equivalent Melanopic Lux (EML), Circadian Action Factor (CAF), sunlight illuminance (Lux) and color temperature (CT) change. In the embodiment of the invention, the portable communication device 630 can go to the "cloud database of multispectral light-emitting devices" in the cloud, select a 5700K multispectral formula with geographical coordinates, and also obtain the circadian rhythm parameter setting of the circadian rhythm in the morning (08:00-12:00) through step 730. At this time, the portable communication device 630 may select to directly control the lamp group 621 to perform the lighting of the 5700K multispectral recipe and the circadian rhythm parameters. In addition, the portable communication device 630 may also select to transmit the 5700K multispectral recipe and the circadian rhythm parameter settings to the ambient light sensor 650, which drives the lamp group 621 to lighting.

Next, it is necessary to further confirm whether the lighting result of the lighting field end 620 can reach the light lighting scenario of the set circadian rhythm lighting, as shown in step 740. When confirming whether the lighting field end 620 can reach the light lighting scenario of its set circadian rhythm lighting, you can choose to measure the Equivalent Melanopic Lux (EML) in the lighting field end 620 through a handheld spectrometer (not shown in the figure) or a full beam integrating sphere (not shown in the figure), as shown in step 7410, and the Circadian Action Factor (CAF), as shown in step 7420. Among them, the hand-held spectrometer measures at a specific range from the lamp group 621, and the full beam integrating sphere places the lamp group 621 in a specific space for measurement.

Then, the software calculates the values of Equivalent Melanopic Lux (EML) and Circadian Action Factor (CAF) respectively, and records the values. Therefore, in this embodiment, it is necessary to confirm whether the Equivalent Melanopic Lux (EML) is above 200 and whether the value of Circadian Action Factor (CAF) is above 0.86. In addition, in the lighting field end 620, the light sensor on the ambient light sensor 650 will also provide whether the detected lighting is greater than 500lux and whether the color temperature is greater than 5700K. Meanwhile, in the embodiment of the invention, the ambient light sensor 650 will further control the scenario parameters, for example, the direct lighting lamp group 520 in the scenario parameters provides 80-100% of the light, while the indirect lighting lamp group 530 provides 0-20% of the light. These measured and detected values are transmitted back to the portable communication device 630 or the management control module 611.

Next, as shown in step 750, it is determined whether the lighting of the lighting field end 620 or the space 500 meets the needs of the circadian rhythm lighting scenario. The determination process may be performed by the portable communication device 630 or the management control module 611. For example, when the portable communication device 630 or the management control module 611 determines that the circadian rhythm parameters of the lighting field end 620 or the circadian rhythm lighting scenario of the space 500 have reached the set value, as shown in step 770, the portable communication device 630 or the management control module 611 in the lighting system 600 transmits the multispectral lighting parameters and circadian rhythm parameters at this time to the cloud 610 or the switching device 640. To establish a multispectral circadian rhythm lighting scenario database. Obviously, the parameter information recorded in the multispectral circadian rhythm lighting scenario database established in step 770 includes the multispectral lighting parameters such as spectrum, light intensity, flicker frequency and color rendering index (Ra) of the lamp group (e.g., LED lamps), as well as various circadian rhythm parameters in Table 6 or Table 7. The circadian rhythm parameters include circadian rhythm lighting parameters and circadian rhythm scenario parameters.

When the portable communication device 630 or the management control module 611 determines that the multispectral lighting parameters or the circadian rhythm parameters of the lighting field end 620 or the circadian rhythm lighting scenario of the space 500 do not reach the set value, it will return to step 720, and the ambient light sensor 650 will adjust the lighting parameters of the lamp group 621 or the lighting ratio of the scenario parameters, then, the portable communication device 630 or the management control module 611 determines whether the multispectral lighting parameters or the circadian rhythm parameters of the circadian rhythm lighting scenario at the lighting field end 620 or the space 500 have reached the set value, and runs until each time differentiated physiological section in Table 6 or Table 7 can reach the set value, and after a complete multispectral circadian rhythm lighting scenario database has been established. The lighting system 600 can fully confirm that the lighting field end 620 or the space 500 can provide the circadian rhythm parameters of an effective circadian rhythm lighting scenario. It should be further emphasized that when using hand-held spectrometer or full beam integrating sphere to measure circadian rhythm lighting parameters, it requires testing space, time, and cost to complete the measurement. Therefore, it is necessary to distinguish the cycle of corresponding circadian rhythm according to the time of 24 hours a day, measure the lighting environment parameters of various circadian rhythm lighting in advance, and establish a circadian rhythm lighting database. The method for constructing a multispectral circadian rhythm lighting scenario database of the invention can be commercialized through the collection of massive data and the learning of AI. In the preferred embodiment of the invention, the adjustment of the circadian rhythm parameters is basically based on the control of the circadian rhythm scenario parameters of the direct lighting lamp group 520 and the indirect lighting lamp group 530, because the lighting field end 620 may be affected by other surrounding light sources.

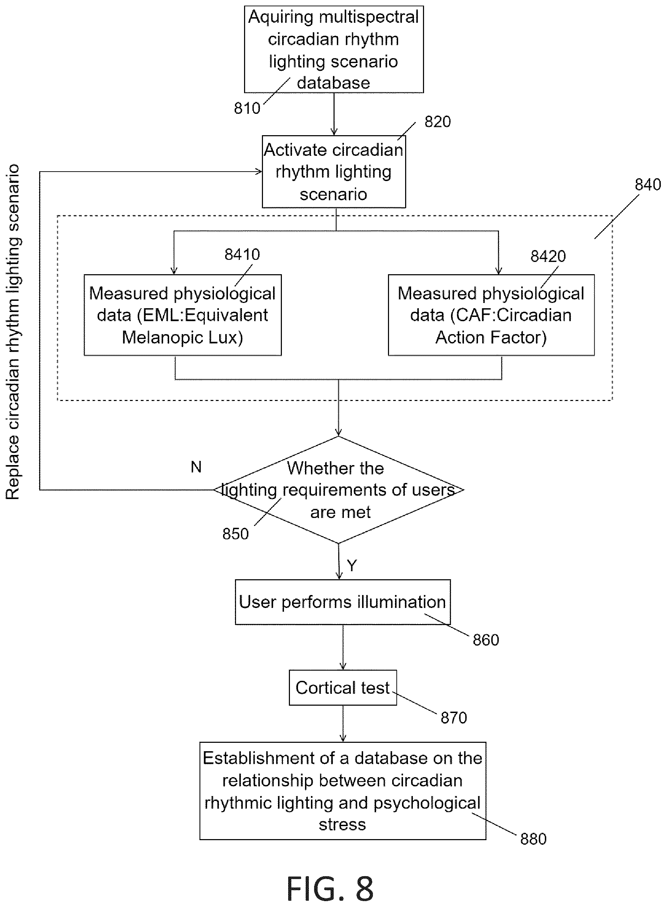

After confirming that the lighting field end 620 or the space 500 can provide the multispectral lighting parameters and circadian rhythm parameters of an effective circadian rhythm lighting scenario, the invention can provide the lighting field end 620 and the lamp group 621 of an effective circadian rhythm lighting scenario. Next, the invention provides a method for establishing a database of the correlation between multispectral circadian rhythm lighting and psychological stress, as shown in FIG. 8. According to the method in FIG. 8, we can know that the status of the illuminator can be judged by the index of psychological pressure after the illuminator is illuminated by an effective circadian rhythm lighting scenario.

First, as shown in step 810, the "multispectral circadian rhythm lighting scenario database" can be retrieved from the cloud 610 through the network (e.g., AIoT) through the portable communication device 630. Then, the circadian rhythm parameter at a specific time can be selected through the portable communication device 630. Then, the lamp group 621 can be driven directly through the APP in the portable communication device 630 to provide the circadian rhythm parameter setting at which time. The obtained "multispectral circadian rhythm lighting scenario database" can also be provided to the ambient light sensor 650 through the portable communication device 630, and the ambient light sensor 650 drives the circadian rhythm parameters at which time to provide lighting. Among them, the parameter information recorded in the above multispectral circadian rhythm lighting scenario database includes multispectral lighting parameters such as spectrum, light intensity, flicker frequency and color rendering (Ra) of lamp group 621 (such as LED lamps), as well as various circadian rhythm parameters in Table 6 or Table 7. Among them, the circadian rhythm parameters include circadian rhythm lighting parameters and circadian rhythm scenario parameters.

Next, as shown in step 820, "start circadian rhythm lighting scenario lighting" is to start the lamp group 621 to illuminate according to the set multispectral lighting parameters and circadian rhythm parameters according to the actual time differentiation through the application program (APP) in the portable communication device 630, so that the lighting at the lighting field end 620 or the space 500 reaches the set values of Equivalent Melanopic Lux (EML), Circadian Action Factor (CAF), sunlight illuminance (Lux) and color temperature change. In the embodiment of the invention, the portable communication device 630 can select the circadian rhythm parameter setting of the circadian rhythm in the morning (08:00-12:00) through the network (e.g., AIoT) to the "multispectral circadian rhythm lighting scenario database" of the cloud 610. At this time, the portable communication device 630 may select to directly control the lighting of the lamp group 621 to perform the circadian rhythm parameters. In addition, the portable communication device 630 may also choose to transmit the circadian rhythm parameter settings to the ambient light sensor 650, and then the ambient light sensor 650 drives the lamp group 621 to illuminate. It is obvious that the present invention does not limit that the portable communication device 630 or the ambient light sensor 650 drives the lamp group 621 for lighting.

Then, as shown in step 840, the invention can quickly detect whether the multispectral lighting parameters and circadian rhythm parameters of the lighting field end 620 have reached the set value through the light sensing device on the ambient light sensor 650. In particular, the rapid detection of the ambient light sensor 650 described in this embodiment is because the ambient light sensor 650 detects the multispectral lighting parameters and circadian rhythm parameters with a period of 10 seconds, and sends the detection results to the portable communication device 630 or the management control module 611. Therefore, it can quickly determine whether the multispectral lighting parameters and circadian rhythm parameters of the lighting field end 620 or the space 500 have reached the set values. Therefore, this embodiment can carry out commercial operation.

Next, as shown in step 850, when the portable communication device 630 or the management control module 611 determines that the multispectral lighting parameters and circadian rhythm parameters have reached the set values, go to step 860 and let the illuminator receive lighting for a period of time. Then, as shown in step 870, the user is tested for cortisol to determine the change of pressure cortisol, confirm that the user has met the lighting needs of the circadian rhythm lighting scenario. For example, when the illuminator receives the lighting of the multispectral lighting parameters and circadian rhythm parameters for a certain time in the morning (08:00-12:00), then go to step 870, immediately sample the blood or saliva of the illuminator through the blood or saliva of the control illuminator, and detect the content of bioactive related to stress hormone in the blood or saliva of the illuminator. In the embodiment of the invention, the detection is mainly aimed at cortisol. For example, when the cortisol index value of the illuminator is between 3.7-19.4, it means that the illuminator has met the lighting needs of the circadian rhythm lighting scenario. In the embodiment of the invention, the cortisol index value of the control light is tested to judge the change of the cortisol of the pressure hormone, and confirm that the user has met the lighting requirements of the circadian rhythm lighting scenario. In other words, it is confirmed that the change of cortisol index value of the user's stress hormone is within the set range when the user meets the requirements of circadian rhythm lighting.

Finally, through step 880, the present invention records the test value of cortisol of the illuminator in the database of the relationship between circadian rhythm lighting and psychological stress, so that the present invention can build a multispectral circadian rhythm lighting scenario database, collect massive data, and then learn from AI, it can more accurately judge whether the illuminator has met the lighting needs of the circadian rhythm lighting scenario according to the change of the cortisol index value of the illuminator, so as to achieve commercialized application. Obviously, the information recorded in the multispectral circadian rhythm lighting scenario database established in step 880 includes the multispectral lighting parameters such as spectrum, light intensity, flicker frequency and color rendering (Ra) of the lamp group (e.g., LED lamps), various circadian rhythm parameters and cortisol index values in Table 6 or Table 7.

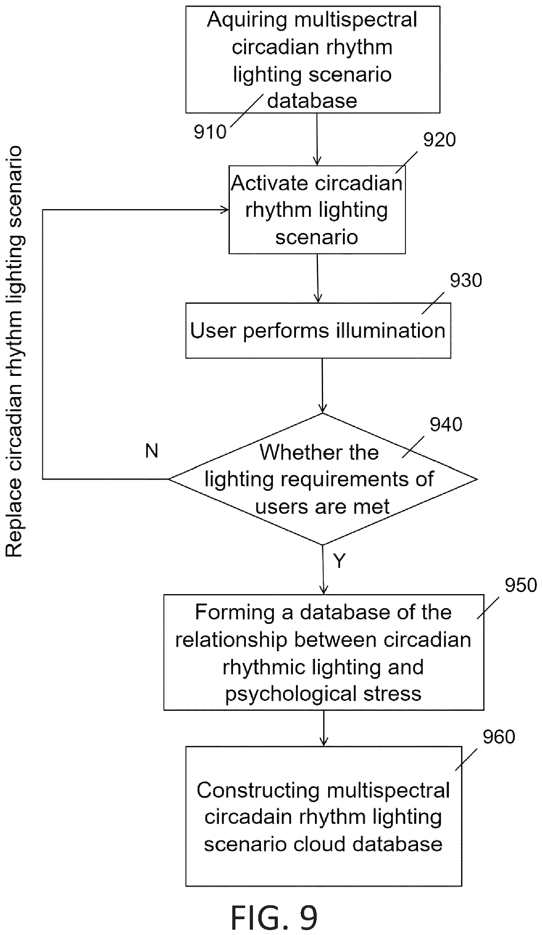

Next, the invention further provides a simple lighting method for multispectral circadian rhythm lighting scenarios, as shown in FIG. 9. First, as shown in step 910, it is necessary to confirm that the lighting field end 620 or the space 500 can provide the multispectral lighting parameters and various circadian rhythm parameters of an effective circadian rhythm lighting scenario. "The portable communication device 630 retrieves the" multispectral circadian rhythm lighting scenario database "from the network (e.g., AIoT) to the cloud 610, so that the invention can provide the lighting field end 620 and the lamp group 621 of an effective circadian rhythm lighting scenario. Among them, the parameter information recorded in the above multispectral circadian rhythm lighting scenario database includes multispectral lighting parameters such as spectrum, light intensity, flicker frequency and color rendering (Ra) of lamp group 621 (such as LED lamps), as well as various circadian rhythm parameters in Table 6 or Table 7. Among them, the circadian rhythm parameters include circadian rhythm lighting parameters and circadian rhythm scenario parameters.

Next, as shown in step 920, the time at which the circadian rhythm parameter setting is provided by the lamp group 621 is selected and driven by the APP of the portable communication device 630. Of course, in step 920, the invention can also choose to provide the obtained "multispectral circadian rhythm lighting scenario database" to the ambient light sensor 650 through the portable communication device 630, and the ambient light sensor 650 drives the multispectral lighting parameters and the circadian rhythm parameters at which time to illuminate. The invention is not limited to this.

Then, as shown in step 930, the illuminator is allowed to receive lighting for a period of time. Then, go to step 940 to judge whether the cortisol index value of the illuminator is within the set range. For example, when the illuminator receives the lighting of multispectral lighting parameters and circadian rhythm parameters for a certain time in the morning (08:00-12:00), and then samples the blood and saliva of the control illuminator, the content of bioactive related to stress hormone in the blood or saliva of the illuminator is detected. In the embodiment of the invention, the detection is mainly aimed at cortisol. For example, when the adrenal cortisol test value of the illuminator is between 3.7 and 19.4, it means that the illuminator has met the lighting requirements of the circadian rhythm lighting scenario. In the embodiment of the invention, the test is performed by comparing the cortisol of the light subject, so as to judge the change of the cortisol of the stress hormone and confirm that the user has met the lighting requirements of the circadian rhythm lighting scenario, as shown in step 950.

Then, as shown in step 950, after confirming that the user has met the lighting needs of the circadian rhythm lighting scenario, go to step 960 to record the indicator value of the adrenal cortisol test of the illuminator into the circadian rhythm lighting and psychological stress correlation database to form a "multispectral circadian rhythm lighting scenario cloud database".

When it is confirmed that the user fails to meet the lighting requirements of the circadian rhythm lighting scenario, for example, when the indicator value of the adrenal cortisol test of the illuminator is not within the range of the indicator values listed in Table 6 or Table 7, it will return to step 920, and select a new multispectral lighting parameter through the APP of the portable communication device 630, the portable communication device 630 or the ambient light sensor 650 may drive the lamp group 621 to illuminate the illuminator with the new multispectral lighting parameters and the circadian rhythm parameters at which time, as shown in step 930. After the irradiator irradiates for a period of time, go to step 940 to determine whether the cortisol index value of the irradiator is within the set range. Obviously, steps 920 to 940 can be repeated until it is judged that the cortisol index value of the illuminator is within the set range. Then, as shown in steps 950 and 960, the index value of the adrenal cortisol test of the illuminator can be recorded in the circadian rhythm lighting and psychological stress correlation database to form a "multispectral circadian rhythm lighting scenario cloud database".

Through the above-mentioned "cloud database of multispectral circadian rhythm lighting scenario", the invention can more accurately judge whether the illuminator has met the lighting needs of circadian rhythm lighting scenario according to the change of cortisol index value of the illuminator through the collection of massive data and the learning of AI, to achieve commercialized application. Obviously, the information recorded in the multispectral circadian rhythm lighting scenario database established in step 960 includes the multispectral lighting parameters such as spectrum, light intensity, flicker frequency and color rendering (Ra) of the lamp group (such as LED lamps), various circadian rhythm parameters in Table 6 or Table 7, and cortisol index values.

Finally, it should be emphasized again that the above description is only a better embodiment of the invention and is not intended to limit the scope of the invention. At the same time, the above description can be understood and implemented by those who have ordinary knowledge in the relevant technical field. Therefore, other equivalent changes or modifications not divorced from the concepts disclosed in the invention should be included in the scope of patent claims of the invention.

## Claims

1. A lighting system with a multispectral circadian rhythm lighting scenario, the lighting system is configured in a space and is composed of a portable communication device, a management control module, a cloud, an ambient light sensor and a light-emitting device, the characterized is in that the execution steps of the lighting system include:
acquiring a multispectral circadian rhythm lighting scenario database, wherein the portable communication device downloads the multispectral circadian rhythm lighting scenario database from internet to the cloud;
starting the lighting of the circadian rhythm lighting scenario, the portable communication device selects a multispectral lighting parameter and a circadian rhythm parameter from the multispectral circadian rhythm lighting scenario database to active the light-emitting device to perform an illumination on an illuminator;
performing adrenal cortisol test is to obtain the psychological stress index value of the illuminator after testing the blood or saliva of the illuminator; and
establishing a multispectral circadian rhythm lighting scenario and psychological stress correlation database, and the portable communication device or the management control module transmits the circadian rhythm parameters and the psychological stress index values to the cloud.

2. The lighting system with multispectral circadian rhythm lighting scenario according to claim 1, wherein the circadian rhythm parameters include circadian rhythm lighting parameters and circadian rhythm scenario parameters.

3. The lighting system with multispectral circadian rhythm lighting scenario according to claim 1, wherein the multispectral lighting parameters includes spectrum, light intensity, flicker frequency and color rendering (Ra) of the light-emitting device.

4. The lighting system with multispectral circadian rhythm lighting scenario according to claim 2, wherein the circadian rhythm lighting parameters include Equivalent Melanopic Lux (EML), Circadian Action Factor (CAF), sunlight illuminance, color temperature change.

5. The lighting system with multispectral circadian rhythm lighting scenario according to claim 2, wherein the circadian rhythm scenario parameters are composed of the light illuminated by the direct lighting device and the indirect lighting device.

6. The lighting system with a multispectral circadian rhythm lighting scenario according to claim 5, wherein the direct light device is a recessed lamp, a pendent lamp, and a ceiling lamp.

7. The lighting system with a multispectral circadian rhythm lighting scenario according to claim 5, wherein the light of the indirect lighting device is formed by reflecting the light of the lighting device by the wall.

8. The lighting system with a multispectral circadian rhythm lighting scenario according to claim 1, wherein the psychological stress index value is a cortisol index value.

9. The lighting system with a multispectral circadian rhythm lighting scenario according to claim 1, wherein the information in the establishment of the associated database includes psychological stress index values and circadian rhythm parameters.

10. A lighting system with a multispectral circadian rhythm lighting scenario, the lighting system is configured in a space and is composed of a portable communication device, a management control module, a cloud, an ambient light sensor and a light-emitting device, the **characterized in that** the execution steps of the lighting system include:
acquiring a multispectral circadian rhythm lighting scenario database, wherein the portable communication device downloads the multispectral light-emitting device cloud database through internet to the cloud;
starting the lighting of the circadian rhythm lighting scenario, the portable communication device selects a multispectral lighting parameter and a circadian rhythm parameter from the multispectral circadian rhythm lighting scenario database, transmits the parameters to the ambient light sensor to active the light-emitting device to perform an illumination on an illuminator;
performing adrenal cortisol test to obtain the psychological stress index value of the illuminator after sampling and testing the blood or saliva of the illuminator; and
establishing a multispectral circadian rhythm lighting scenario and psychological stress correlation database, and the portable communication device or the management control module transmits the circadian rhythm parameters and the psychological stress index values to the cloud.

11. A lighting method with multispectral circadian rhythm lighting scenario is to configure a light-emitting device and a lighting system in a space, wherein the lighting system is composed of a portable communication device, a management control module, a cloud and an ambient light sensor, the characterized is in that the lighting method comprises:
acquiring a multispectral circadian rhythm lighting scenario database, wherein the portable communication device downloads the multispectral circadian rhythm lighting scenario database from internet to the cloud;
starting the lighting of the circadian rhythm lighting scenario, the portable communication device selects a multispectral lighting parameter and a circadian rhythm parameter from the multispectral circadian rhythm lighting scenario database to start the light-emitting device to perform an illumination on an illuminator;
performing adrenal cortisol test to obtain the psychological stress index value of the illuminator after testing the blood or saliva of the illuminator; and
establishing a multispectral circadian rhythm lighting scenario and psychological stress correlation database, and the portable communication device or the management control module transmits the circadian rhythm parameters and the psychological stress index values to the cloud.

12. The lighting method with multispectral circadian rhythm lighting scenario according to claim 11, wherein the circadian rhythm parameters include: circadian rhythm lighting parameters and circadian rhythm scenario parameters.

13. The lighting method with multispectral circadian rhythm lighting scenario according to claim 11, wherein the multispectral lighting parameters includes spectrum, light intensity, flicker frequency and color rendering (Ra) of the light-emitting device.

14. The lighting method with multispectral circadian rhythm lighting scenario according to claim 12, wherein the circadian rhythm lighting parameters include Equivalent Melanopic Lux (EML), Circadian Action Factor (CAF), sunlight illuminance, color temperature change.

15. The lighting method with multispectral circadian rhythm lighting scenario according to claim 12, wherein the circadian rhythm scenario parameter is composed of the light illuminated by the direct lighting device and the indirect lighting device.

16. The lighting method with a multispectral circadian rhythm lighting scenario according to claim 15, wherein the direct light device is a recessed lamp, a pendent lamp, and a ceiling lamp.

17. The lighting method with a multispectral circadian rhythm lighting scenario according to claim 15, wherein the light of the indirect lighting device is formed by reflecting the light of the lighting device by the wall.

18. The lighting method with multispectral circadian rhythm lighting scenario according to claim 11, wherein the psychological stress index value is a cortisol index value.

19. The lighting method with a multispectral circadian rhythm lighting scenario according to claim 11, wherein the information in the database for establishing the relationship between the multispectral circadian rhythm lighting scenario and psychological stress includes: psychological stress index value and circadian rhythm parameters.

20. The lighting method with a multispectral circadian rhythm lighting scenario according to claim 11, wherein when starting the lighting of the circadian rhythm lighting scenario, the portable communication device selects a multispectral lighting parameter and a section parameter from the multispectral circadian rhythm lighting scenario database, and transmitting the multispectral lighting parameter and the circadian rhythm parameter to the ambient light sensor to active the light-emitting device for lighting.
